# EUROPEAN PATENT APPLICATION

(11) **EP 2 409 763 A1**
(43) Date of publication of application: **25.01.2012**
(21) Application number: 10753578.3
(22) Date of filing: 18.03.2010
(51) Int. Cl.: B01J 35/02, A61L 9/00, A61L 9/01, B01D 53/86, B01J 37/02

(54) **PHOTOCATALYST MEMBER**

(30) Priority: 18.03.2009 JP 2009065849
(71) Applicant: Toto Ltd., Fukuoka 802-8601 (JP)
(72) Inventor: HAYAKAWA, Makoto, Kitakyushu-shi Fukuoka 802-8601 (JP); KATAOKA, Kyoko, Kitakyushu-shi Fukuoka 802-8601 (JP); KITAZAKI, Satoru, Kitakyushu-shi Fukuoka 802-8601 (JP); KAMESHIMA, Junji, Kitakyushu-shi Fukuoka 802-8601 (JP); KANNO, Mitsuyoshi, Kitakyushu-shi Fukuoka 802-8601 (JP)
(74) Representative: Grünecker, Kinkeldey, Stockmair & Schwanhäusser
(86) International application number: PCT/JP2010/054671
(87) International publication number: WO 2010/107087

(57) **Abstract**

Disclosed is a photocatalyst member that, even when used in a form where warm water resistance and acid resistance are required, can exert a photocatalytic decomposition ability over a long period of time. The member having a photocatalytic decomposition ability comprises: a base material; and photocatalytic metal oxide particles immobilized on the base material, the base material having a surface treated with silicification flame treatment, the photocatalytic metal oxide particles are immobilized with a silicon-type binder on the base material on the surface treated with silicification flame treatment.

## Description

### TECHNICAL FIELD

The present invention relates to a photocatalyst member that comprises a photocatalyst layer having excellent photocatalyst decomposition ability and adhesion to a base material and is suitable particularly for applications, for example, in exterior materials and interior materials of buildings, which includes metal panels, resin panels, clad steel plates, resin-coated steel plates, resin-coated ceramic building materials.

### BACKGROUND ART

Photocatalysts such as titanium oxide have recently been used in many applications such as exterior and interior materials of buildings. For exterior applications, coating a photocatalyst on the surface of a base material can allow an ability of decomposing harmful substances such as NOx and SOx and a self-cleaning ability to be imparted by utilizing photo energy. Also for interior applications, an ability of decomposing harmful substances such as VOCs (volatile organic compounds) and antimicrobial/fungicide/antivirus ability to be imparted by utilizing photo energy.

In order to satisfactorily exert the photocatalytic decomposing ability, it is necessary to immobilize photocatalyst particles on the outermost layer so that active oxygen species having a short life generated by a photoexcitation reaction of photocatalyst particles are satisfactorily brought into contact with substances to be decomposed such as a harmful gas. When an attempt to immobilize photocatalyst particles directly on the base material is made, the adhesion is sometimes unsatisfactory depending upon the type of the material. For example, the following methods have hitherto been adopted from the viewpoint of ensuring satisfactory adhesion. (1) The surface of the base material is roughened by discharge treatment, etching or the like, and photocatalyst particles are immobilized thereon. (2) The photocatalyst particles are immobilized by incorporating a resin having good adhesion to the base material (for example, acrylic resins). (3) A primer layer is formed on the surface of the base material, and photocatalyst particles are immobilized thereon.

For using the photocatalyst technology under specific conditions, however, a method for immobilizing photocatalyst particles on the base material with satisfactory adhesion has been still desired. In particular, there is need of a method for preparing photocatalyst members that have excellent adhesion to the photocatalyst particles and the base material after a warm water resistance test and an acid resistance test while satisfactorily exerting a photocatalytic decomposing ability, that is, there is need of photocatalyst members that can satisfactorily exert an ability of photocatalytically decomposing substances to be decomposed such as harmful gases over a long period of time under the condition that warm water resistance and acid resistance are required.

### [Citation List]

### [Patent Literature]

[PLT 1] JP 2004-225430A
[PLT 2] JP H10(1998)-67873A
[PLT 3] JP H9(1997)-234375A
[PLT 4] JP 2005-105053A
[PLT 5] JP 2001-47584A
[PLT 6] JP 2003-238710A

### SUMMARY OF THE INVENTION

The present inventors have now found that a combination of silicification flame treatment of the surface of the base material with the use of a silicon-type binder can allow photocatalyst particles to be strongly immobilized on the base material, especially photocatalyst particles to be sustainably immobilized on the base material even when the member is used under specific conditions.
Accordingly, an object of the present invention is to provide a member and a process for preparing thereof, the member comprising photocatalyst particles immobilized strongly on a base material and possess a photocatalytic decomposition ability, especially a member that can allow photocatalyst particles to be immobilized sustainably and strongly on a base material and thus can allow the photocatalytic decomposition ability to be maintained even when the member is used under specific conditions. In particular, the present invention is to provide a photocatalyst member that has excellent adhesion between photocatalyst particles and a base material after a warm water resistance test and an acid resistance test while satisfactorily exerting a photocatalyst-derived decomposition ability, that is, a photocatalyst member that can allow an ability of decomposing substances to be decomposed such as harmful gas by a photocatalyst over a long period of time even in the form of use where warm water resistance and acid resistance are required.

According to the present invention, there is provided a photocatalyst member having a photocatalytic decomposition ability, the member comprising: a base material; and photocatalytic metal oxide particles immobilized on the base material, the base material having a surface treated with silicification flame treatment, the photocatalytic metal oxide particles are been immobilized with a silicon-type binder on the surface of the base material treated with silicification flame treatment.
According to the present invention, there is also provided a method for preparing a photocatalyst member having a photocatalytic decomposition ability, the member comprising: a base material; and photocatalytic metal oxide particles immobilized on the base material, the method comprising: treating at least one surface of a base material with silicification flame treatment; and immobilizing photocatalytic metal oxide particles with a silicon-type binder on the treated surface of the base material.

### PREFERRED EMBODIMENT OF THE INVENTION

### Photocatalyst member

The photocatalyst member according to the present invention has a basic construction comprising a base material having a surface treated with silicification flame treatment and photocatalytic metal oxide particles immobilized on the treated surface of the base material with the aid of a silicon-type binder and possesses a photocatalytic decomposition ability. The photocatalyst member according to the present invention possesses excellent adhesion between photocatalyst particles and the base material after warm water resistance tests and acid resistance tests while satisfactorily exerting a photocatalytic decomposition ability and more specifically can satisfactorily exert a photocatalytic decomposition ability over a long period of time even in the form of use where warm water resistance and acid resistance are required. The reason why such excellent adhesion can be realized is believed to reside in that the silicon-type binder is satisfactorily spread while wetting, and, at the same time, silanol groups present within the binder are strongly bound to silanol groups present on the surface treated with the silicification flame treatment, and, further, the binder get used to the photocatalyst particles. However, it should be noted that the explanation is hypothetical, and the present invention is not limited to the hypothesis.

### Silicification flame treatment

In the present invention, the silicification flame treatment refers to a method that comprises gasifying and incorporating a silicon atom-containing modifier compound into a combustion gas and subjecting the surface of the base material to flame treatment using the combustion gas containing the modifier compound.

In the present invention, the silicon atom-containing modifier compound preferably includes compounds having a boiling point of 10 to 100°C and as specific examples preferably to be used includes tetramethylsilane, tetraethylsilane, 1,2-dichlorotetramethylsilane, 1,2-dichlorotetraethylsilane, 1,2-diphenyltetramethylsilane, 1,2-diphenyltetraethylsilane, 1,2,3-trichlorotetramethylsilane, 1,2,3-triphenyltetramethylsilane, and dimethyldiethylsilane.

In a preferred embodiment of the present invention, flammable gases such as propane and carrier gases can be incorporated into the combustion gas.

### Photocatalytic metal oxide particles

In the present invention, examples of photocatalytic metal oxide particles include anatase form of titanium oxide, rutile form of titanium oxide, brookite form of titanium oxide, tin oxide, zinc oxide, strontium titanate, tungsten oxide, and cerium oxide. Among them, a mixture of two or more of them may also be used. Further, the photocatalytic metal oxide particles include metal oxide particles of the type described above into which metals such as platinum, gold, silver, silver oxide, copper, cuprous oxide, cupric oxide, and iron, nitrogen, or anions such as fluorine are doped so that excitation can be made by visible light.

The photocatalyst particles preferably have an average particle diameter of not less than 10 nm and less than 100 nm, more preferably not less than 10 nm and not more than 60 nm. The average particle diameter is calculated as a number average value determined by measuring the length of randomly selected 100 particles in a visual field at a magnification of 200000 times under a scanning electron microscope.

### Silicon-type binder

In the present invention, suitable silicon-type binders preferably to be used include silicones, alkylsilicates, and silyl group-containing vinyls comprising a di- to tetra-functional silane as a monomer unit. Silicones suitable for use herein include modified silicones such as phenyl-containing silicones, acryl-containing silicones, epoxy-containing silicones, amino-containing silicones, hydro-containing silicones, and vinyl-containing silicones, and alkyl-containing silicones. Suitable forms of binders include solutions and emulsions.

### Base material

In the present invention, the base material is not particularly limited, and any of metals, ceramics, resins, or mixtures, composites, or laminates thereof is usable. In a preferred embodiment of the present invention, metals or hydrophobic resins exert a more significant effect. For these base materials, in conventional methods such as corona discharge, difficulties are encountered in simultaneously satisfying photocatalyst properties and adhesion between the photocatalyst particles and the base material after warm water resistance test/acid resistance test of resin, whereas, in the present invention, the photocatalyst properties and the adhesion can be simultaneously satisfied.

Metal base materials suitable for use herein include aluminum, stainless steels, and copper alloys. Hydrophobic resin base materials suitable for use herein include unsaturated polyester resins, vinyl chloride resins, polyethylene resins, polypropylene resins, polystyrene resins, melamine resins, ABS resins, polyurethane resins, epoxy resins, fluororesins, acrylic resins, polycarbonate resins, and silicone resins.

In one embodiment of the present invention, there is provided a photocatalyst member having a photocatalytic decomposition ability, the member comprising: a base material; and photocatalytic metal oxide particles immobilized on the base material, the surface of the base material with the photocatalytic metal oxide particles immobilized thereon having been treated with silicification flame treatment, a layer containing both the photocatalytic metal oxide particles and the silicon-type binder having been formed on the surface treated with silicification flame treatment.

The member can be formed by successively performing the steps of: treating at least one surface of a base material with silicification flame treatment; coating photocatalytic metal oxide particles and a silicon-type binder onto the base material on its surface treated with silicification flame treatment, and curing the silicon-type binder.

The content of the silicon-type binder in the layer containing the photocatalytic metal oxide particles and the silicon-type binder is preferably 10 to 90% by mass from the viewpoint of simultaneously satisfying an ability of photocatalytically decomposing substances to be decomposed such as harmful gases and adhesion between the photocatalyst particles and the base material after the warm water resistance test/acid resistance test.

At least one metal selected from the group consisting of vanadium, iron, cobalt, nickel, palladium, zinc, ruthenium, rhodium, copper, silver, platinum, and gold and/or a metal compound comprising the metal may be added to the layer containing the photocatalytic metal oxide particles and the silicon-type binder from the viewpoint of developing a higher photocatalyst ability.

Further, the layer containing the photocatalytic metal oxide particles and the silicon-type binder may include inorganic oxide particles to enhance the probability of contact between the photocatalyst and the substances to be decomposed such as harmful gas. The inorganic oxide particles suitable for use herein include, for example, silica, alumina, zirconia, ceria, yttria, tin oxide, iron oxide, manganese oxide, nickel oxide, cobalt oxide, hafnia, barium titanate, calcium silicate, aluminum borate, potassium titanate, zeolite, apatite, and calcium phosphate.

The inorganic oxide particles preferably have an average particle diameter of more than 5 nm and not more than 50 nm, more preferably not less than 10 nm and not more than 40 nm, from the viewpoint of forming a film having high sliding abrasion resistance while enhancing the probability of contact between the photocatalyst and the substances to be decomposed such as harmful gas. The average particle diameter is calculated as a number average value determined by measuring the length of 100 randomly selected particles in a visual field at a magnification of 200,000 times under a scanning electron microscope. The shape of the particles is most preferably spherical and may also be substantially circular or elliptical. In this case, the length of the particles is approximately calculated as ((major axis + minor axis)/2).

The thickness of the dried layer containing the photocatalytic metal oxide particles and the silicon-type binder i s not particularly limited, but is preferably 0.1 µm to 50 µm, more preferably 0.1 µm to 10 µm, from the viewpoint of simultaneously satisfying an ability of photocatalytically decomposing substances to be decomposed such as harmful gases and crack resistance.

The layer containing the photocatalytic metal oxide particles and the silicon-type binder may contain extender pigments, coloring pigments and the like as optional ingredients.

Examples of suitable extender pigments include titanium oxide whiskers, calcium carbonate whiskers, aluminum borate whiskers, potassium titanate whiskers, mica, and talc.

Coloring pigments suitable for use herein include, for example, inorganic coloring pigments such as titanium oxide white, zinc oxide white, iron oxide, carbon black, spinel green, iron oxide red, cobalt aluminate, or ultramarine, and organic coloring pigments such as phthalocyanine pigments, benzimidazolone pigments, isoindoline pigments, azo pigments, anthraquinone pigments, quinophthalone pigments, anthrapyridine pigments, quinacridone pigments, toluidine pigments, pyrathrone pigments, and perylene pigments.

In another embodiment of the present invention, there is provided a photocatalyst member having a photocatalytic decomposition ability, the member comprising: a base material; and photocatalytic metal oxide particles immobilized on the base material, the base material on its surface, on which the photocatalytic metal oxide particles are immobilized, being treated with silicification flame treatment, a b inder layer containing a silicon-type binder being formed on the surface treated with silicification flame treatment, a layer containing the photocatalytic metal oxide particles being formed on the binder layer.

The member may be formed by the steps of: treating the surface of a base material with silicification flame treatment, then coating a silicon-type binder onto the surface treated with silicification flame treatment, coating a coating liquid containing photocatalytic metal oxide particles, curing the silicon-type binder, and drying the coating liquid containing the photocatalytic metal oxide particles.

The layer containing the photocatalytic metal oxide particles may include at least one metal selected from the group consisting of vanadium, iron, cobalt, nickel, palladium, zinc, ruthenium, rhodium, copper, silver, platinum, and gold and/or a metal compound comprising the metals to develop a higher photocatalyst ability.

The layer containing the photocatalytic metal oxide particles may include inorganic oxide particles to enhance the probability of contact between the photocatalyst and the substances to be decomposed such as harmful gas. The inorganic oxide particles suitable for use herein include, for example, silica, alumina, zirconia, ceria, yttria, tin oxide, iron oxide, manganese oxide, nickel oxide, cobalt oxide, hafnia, barium titanate, calcium silicate, aluminum borate, potassium titanate, zeolite, apatite, and calcium phosphate.

The inorganic oxide particles preferably have an average particle diameter of more than 5 nm and not more than 50 nm, more preferably not less than 10 nm and not more than 40 nm, from the viewpoint of forming a film having high sliding abrasion resistance while enhancing the probability of contact between the photocatalyst and the substances to be decomposed such as harmful gas. The average particle diameter is calculated as a number average value determined by measuring the length of 100 randomly selected particles in a visual field at a magnification of 200000 times under a scanning electron microscope. The shape of the particles is most preferably spherical and may also be substantially circular or elliptical. In this case, the length of the particles is approximately calculated as ((major axis + minor axis)/2).

The thickness of the layer containing the photocatalytic metal oxide particles is preferably not less than 0.1 µm and not more than 5.0 µm, more preferably not less than 0.5 µm and not more than 3.0 µm, most preferably not less than 1.0 µm and not more than 2.0 µm. The thickness in the above-defined range can simultaneously realize an ability of photocatalytically decomposing substances to be decomposed such as harmful gases and the transparency of the layer.

For the layer containing the photocatalytic metal oxide particles, ensuring a linear transmittance of the photocatalyst layer of not less than 90%, more preferably not less than 95%, at a wavelength of 550 nm is more preferred. This can prevent loss of color and design of the substrate. Further, there is no possibility that, even when coating on a highly transparent base material lowers the transparency.

The thickness on the dried layer containing the silicon-type binder is not particularly limited, but is preferably less than 50 µm, more preferably 10 µm, from the viewpoint of crack resistance.

The layer containing the silicon-type binder may contain extender pigments, coloring pigments, antialgal agents and the like as optional ingredients.

Examples of suitable extender pigments include titanium oxide whiskers, calcium carbonate whiskers, aluminum borate whiskers, potassium titanate whiskers, mica, and talc.

Coloring pigments suitable for use herein include, for example, inorganic coloring pigments such as titanium oxide white, zinc oxide white, iron oxide, carbon black, spinel green, iron oxide red, cobalt aluminate, or ultramarine, and organic coloring pigments such as phthalocyanine pigments, benzimidazolone pigments, isoindoline pigments, azo pigments, anthraquinone pigments, quinophthalone pigments, anthrapyridine pigments, quinacridone pigments, toluidine pigments, pyrathrone pigments, and perylene pigments.

Antialgal agents suitable for use herein are organic antialgal agents having good compatibility with the resin component in the intermediate layer, and examples thereof include organic nitrogen sulfur compounds, pyrithione compounds, organic iodine compounds, triazine compounds, isothiazoline compounds, imidazole compounds, pyridine compounds, nitrile compounds, thiocarbamate compounds, thiazole compounds, and disulfide compounds.

### EXAMPLES

The present invention is further illustrated by the following Examples that are not intended as a limitation of the invention.

### <Preparation of coated body samples>

Examples 1 to 6 and Comparative Examples 1 to 3 were prepared as follows.

### Example 1:

An aluminum base material was subjected to 0.5-sec silicification flame treatment four times using a fuel gas into which an compressed air with a very small amount of a silicon atom-containing modifier compound having a boiling point of 10 to 100°C had been incorporated. A coating liquid containing an acrylsilicone resin having a silicon content of 10% by mass was coated on the treated surface, and the coating was dried to form a 10 µm-thick intermediate layer. Thereafter, a photocatalyst coating liquid having a solid content of 5.5% by mass with a water dispersion of an anatase form of titanium oxide (average particle diameter: about 50 nm) and a water dispersion-type colloidal silica (average particle diameter: about 30 nm) incorporated therein was spray-coated on the surface of the intermediate layer, and the coating was dried to form a 0.5 µm-thick photocatalyst layer.

### Example 2:

A polypropylene resin base material was subjected to 0.5-sec silicification flame treatment four times using a fuel gas into which an compressed air with a very small amount of a silicon atom-containing modifier compound having a boiling point of 10 to 100°C had been incorporated. A coating liquid containing an acrylsilicone resin having a silicon content of 10% by mass was coated on the treated surface, and the coating was dried to form a 10 µm-thick intermediate layer. Thereafter, a photocatalyst coating liquid having a solid content of 5.5% by mass with a water dispersion of an anatase form of titanium oxide (average particle diameter: about 50 nm) and a water dispersion-type colloidal silica (average particle diameter: about 30 nm) incorporated therein was spray-coated on the surface of the intermediate layer, and the coating was dried to form a 0.5 µm-thick photocatalyst layer.

### Comparative Example 1:

A polypropylene resin base material was subjected to high-frequency corona discharge treatment with a corona surface treatment apparatus using a wire-electrode as an electrode under conditions of a gap between the tip of the electrode and the surface of the sample of 2 mm, a voltage of 26 kV, a frequency of 39 kHz, and a sample feed speed of 4.2 m/min. A coating liquid containing an acrylsilicone resin having a silicon content of 10% by mass was coated on the treated surface, and the coating was dried to form a 10 µm-thick intermediate layer. Thereafter, a photocatalyst coating liquid having a solid content of 5.5% by mass with a water dispersion of an anatase form of titanium oxide (average particle diameter: about 50 nm) and a water dispersion-type colloidal silica (average particle diameter: about 30 nm) incorporated therein was spray-coated on the surface of the intermediate layer, and the coating was dried to form a 0.5 µm-thick photocatalyst layer.

### Example 3:

An ABS resin base material was subjected to 0.5-sec silicification flame treatment four times using a fuel gas into which an compressed air with a very small amount of a silicon atom-containing modifier compound having a boiling point of 10 to 100°C had been incorporated. A coating liquid containing an acrylsilicone resin having a silicon content of 10% by mass was coated on the treated surface, and the coating was dried to form a 10 µm-thick intermediate layer. Thereafter, a photocatalyst coating liquid having a solid content of 5.5% by mass with a water dispersion of an anatase form of titanium oxide (average particle diameter: about 50 nm) and a water dispersion-type colloidal silica (average particle diameter: about 30 nm) incorporated therein was spray-coated on the surface of the intermediate layer, and the coating was dried to form a 0.5 µm-thick photocatalyst layer.

### Example 4:

A PBT resin base material was subjected to 0.5-sec silicification flame treatment four times using a fuel gas into which an compressed air with a very small amount of a silicon atom-containing modifier compound having a boiling point of 10 to 100°C had been incorporated. A coating liquid containing an acrylsilicone resin having a silicon content of 10% by mass was coated on the treated surface, and the coating was dried to form a 10 µm-thick intermediate layer. Thereafter, a photocatalyst coating liquid having a solid content of 5.5% by mass with a water dispersion of an anatase form of titanium oxide (average particle diameter: about 50 nm) and a water dispersion-type colloidal silica (average particle diameter: about 30 nm) incorporated therein was spray-coated on the surface of the intermediate layer, and the coating was dried to form a 0.5 µm-thick photocatalyst layer.

### Example 5:

An acrylic resin base material was subjected to 0.5-sec silicification flame treatment four times using a fuel gas into which an compressed air with a very small amount of a silicon atom-containing modifier compound having a boiling point of 10 to 100°C had been incorporated, whereby the contact angle of the surface of the acrylic resin with water decreased to 12 degrees. A coating liquid containing an acrylsilicone resin having a silicon content of 10% by mass was coated on the treated surface, and the coating was dried to form a 10 µm-thick intermediate layer. Thereafter, a photocatalyst coating liquid having a solid content of 5.5% by mass with a water dispersion of an anatase form of titanium oxide (average particle diameter: about 50 nm) and a water dispersion-type colloidal silica (average particle diameter: about 30 nm) incorporated therein was spray-coated on the surface of the intermediate layer, and the coating was dried to form a 0.5 µm-thick photocatalyst layer.

### Example 6:

An aluminum base material was subjected to 0.5-sec silicification flame treatment four times using a fuel gas into which a compressed air with a very small amount of a silicon atom-containing modifier compound having a boiling point of 10 to 100°C had been incorporated. A photocatalyst coating liquid comprising an anatase form of titanium oxide (average particle diameter: about 50 nm) and an ethyl silicate oligomer dispersed in a mixed solvent composed of alcohol and water was spray-coated on the treated surface, and the coating was dried to form a 0.2 µm-thick photocatalyst layer.

### Comparative Example 2:

A coating liquid containing an acrylsilicone resin having a silicon content of 10% by mass was coated onto the surface of an acrylic resin base material, and the coating was dried to form a 10 µm-thick intermediate layer. Thereafter, a photocatalyst coating liquid having a solid content of 5.5% by mass with a water dispersion of an anatase form of titanium oxide (average particle diameter: about 50 nm) and a water dispersion-type colloidal silica (average particle diameter: about 30 nm) incorporated therein was spray-coated on the surface of the intermediate layer, and the coating was dried to form a 0.5 µm-thick photocatalyst layer.

### Comparative Example 3:

A photocatalyst coating liquid comprising an anatase form of titanium oxide (average particle diameter: about 50 nm) and an ethyl silicate oligomer dispersed in a mixed solvent composed of alcohol and water was spray-coated on the surface of an acrylic resin base material, and the coating was dried to form a 0.2 µm-thick photocatalyst layer

### <Evaluation>

The samples thus obtained were subjected to (1) a photocatalytic decomposition activity test by BLB at 30 W/m² for samples immersed in warm water of 40°C for one week, (2) a tape peel test for samples immersed in warm water of 40°C for one week, (3) a photocatalytic decomposition activity test by BLB at 30 W/m² for samples immersed in a 1% aqueous sulfuric acid solution of 40°C for 3 hr, and (4) a tape peel test for samples immersed in a 1% aqueous sulfuric acid solution of 40°C for 3 hr.
The photocatalytic decomposition activity test was carried out by a method specified in JIS-R1703-2 "Testing method for self-cleaning performance of photocatalytic materials - Part 2: Wet decomposition performance." The results were evaluated as ○ when the decomposition activity index R (nmol/L/min) was not less than 5; and were evaluated as × when the decomposition activity index R (nmol/L/min) was less than 5.
The tape peel test was carried out by a method according to JIS-K-5600. Specifically, cross-cuts were provided on the surface of the coating film, and a pressure-sensitive adhesive tape was applied thereon and was then peeled off at once. The sample was evaluated as ○ when the coating film was not separated; and was evaluated as × when the coating film was separated.
The results of evaluation are shown in Table 1.

**[Table 1]**

| Sample | Decomposition activity after immersion in warm water | Tape peel after immersion in warm water | Decomposition activity after immersion in acid | Tape peel after immersion in acid |
|---|---|---|---|---|
| Example 1 | ○ | ○ | ○ | ○ |
| Example 2 | ○ | ○ | ○ | ○ |
| Example 3 | ○ | ○ | ○ | ○ |
| Example 4 | ○ | ○ | ○ | ○ |
| Example 5 | ○ | ○ | ○ | ○ |
| Example 6 | ○ | ○ | ○ | ○ |
| Comparative Example 1 | | × | | × |
| Comparative Example 2 | | × | | × |
| Comparative Example 3 | | × | | |

## Claims

1. A photocatalyst member having a photocatalytic decomposition ability, the member comprising: a base material; and photocatalytic metal oxide particles immobilized on the base material,
the base material having a surface which is treated with silicification flame treatment,
the photocatalytic metal oxide particles are immobilized with a silicon-type binder on the base material on the surface which is treated with silicification flame treatment.

2. The photocatalyst member according to claim 1, wherein the photocatalytic metal oxide particles and the silicon-type binder are formed as a layer containing both the photocatalytic metal oxide particles and the silicon-type binder on the surface treated with silicification flame treatment.

3. The photocatalyst member according to claim 1, wherein a binder layer containing a silicon-type binder is formed on the surface treated with silicification flame treatment, and a layer containing the photocatalytic metal oxide particles are formed on the binder layer.

4. The photocatalyst member according to any one of claims 1 to 3, wherein the base material is a metallic base material.

5. The photocatalyst member according to claim 4, wherein the base material is a metallic aluminum base material.

6. The photocatalyst member according to any one of claims 1 to 3, wherein the base material is a hydrophobic resin base material.

7. A method for producing a member having a photocatalytic decomposition ability, the member comprising: a base material; and photocatalytic metal oxide particles immobilized on the base material, the process comprising:
treating at least one surface of the base material with silicification flame treatment; and
immobilizing photocatalytic metal oxide particles with a silicon-type binder on the base material on the surface treated with silicification flame treatment.

8. The method according to claim 7, wherein the photocatalytic metal oxide particles are immobilized by forming a layer containing both the photocatalytic metal oxide particles and the silicon-type binder on the base material on the surface treated with silicification flame treatment.

9. The method according to claim 7, wherein a binder layer containing a silicon-type binder is formed on the surface treated with silicification flame treatment, and a layer containing the photocatalytic metal oxide particles is formed on the binder layer.
